# EUROPEAN PATENT SPECIFICATION

(11) **EP 4 351 539 B1**
(45) Date of publication and mention of the grant of the patent: **22.04.2026**
(21) Application number: 22820871.6
(22) Date of filing: 07.06.2022
(51) Int. Cl.: A61K 9/70, A61K 31/137, A61K 31/48, A61L 27/54, A61P 25/24

(54) **METHODS FOR THE PULSED DELIVERY OF BIOACTIVE AGENTS**
VERFAHREN ZUR GEPULSTEN FREISETZUNG BIOAKTIVER WIRKSTOFFE
PROCÉDÉS DE DISTRIBUTION PULSÉE D'AGENTS BIOACTIFS

(30) Priority: 07.06.2021 US 202163197632 P
(43) Date of publication of application: 17.04.2024
(73) Proprietor: University of Mississippi, Oxford, Mississippi 38677 (US); Munivar, Azim, Austin, TX 78704 (US); Puleo, David Allen, Madison, AL 35756 (US)
(72) Inventor: WERFEL, Thomas, Oxford, Mississippi 38655 (US); MUNIVAR, Azim, Austin, Texas 78704 (US); PULEO, David Allen, Madison, AL 35756 (US)
(74) Representative: Berggren Oy
(86) International application number: PCT/US2022/032450
(87) International publication number: WO 2022/261058

(56) References cited:
- WO-A1-2020/157569
- US-A1- 2008 254 095
- US-A1- 2019 307 697
- US-A1- 2020 085 816
- US-A1- 2021 145 777

## Description

### CROSS REFERENCE TO RELATED APPLICATIONS

This application claims the benefit of U.S. Provisional Application No. 63/197,632 filed on June 7, 2021.

### BACKGROUND

Delivery devices capable of delivering bioactive agents over several months is an attractive approach for treating certain types of disease states. For example, depression is a major public health burden domestically and worldwide. Treatment options for patients with major depressive disorder (MDD) consist primarily of psychotherapy and pharmacotherapy. In the latter category specifically, there have been only incremental advances in treatment options until recently when Esketamine, a form of ketamine given as a nasal spray, was approved. WO2020/157569 describes a controlled release tablet formulation comprising psilocybin.

Primary challenges to the treatment of MDD are the relatively low response rates to medication as well as high relapse in a large subset of patients. The most comprehensive study of MDD undertaken was the National Institute of Mental Health-funded Sequenced Treatment Alternatives to Relieve Depression (STAR*D) trial. The trial outlined an algorithmic, sequential treatment approach and thus allows for an estimated likelihood of antidepressant success with subsequent trials. Acute remission rates declined with each additional trial (trial 1, 37%; trial 2, 31%; trial 3, 14%; trial 4, 13%). Correspondingly, the probabilities of remitting and maintaining remission for 1 year decrease with each additional trial (26% for level 1, 14% for level 2, 5% for level 3, and 3% for level 4). Unfortunately, this corresponds to over 43% of patients who fail the first two trials.

Microdosing is one approach for the treatment of depression and other diseases; however, there are limitations to this approach. For example, a major hurdle to furthering the research on 5HT_{2A} agonists such as LSD and psilocybin for the treatment of depression is their current status as Schedule I drugs. Given the potential concerns around diversion or abuse of these compounds, as well as the strict controls on their distribution, dosing is limited to monitored settings. This requires patients to come in for dosing on an every-other (Q48) or every-third day (Q72) schedule for several weeks, which will likely limit patient participation and treatment. Thus, what is needed is the pulsed delivery of bioactive agents to patients such that the patient does not need to be responsible for taking the bioactive agent but is delivered automatically upon administration to the patient.

### SUMMARY

Described herein is a device according to the claims for use in a method for providing the pulsed delivery of a bioactive agent, such as a 5HT_{2A} agonist, to a subject upon administration of a device to the subject. The device is composed of biodegradable polymers such that when administered to the subject the polymers on the surface of the device erode and release the bioactive agent. In one aspect, the device is composed of alternating layers of biodegradable polymers, where every other layer includes the bioactive agent. In a further aspect, one or more of the layers can be composed of a mixture of cellulose acetate phthalate (CAP) and a poloxamer. The layers that do not include the bioactive agent functions as "blanks," which can be used to control the release rate of the bioactive agent. In a still further aspect, one or more sides of the device can be coated with a biodegradable and/or biocompatible polymer such that the bioactive agent is released from only one side of the device.

Other systems, methods, features, and advantages of the present disclosure will be or become apparent to one with skill in the art upon examination of the following drawings and detailed description. It is intended that all such additional systems, methods, features, and advantages be included within this description, be within the scope of the present disclosure, and be protected by the accompanying claims. In addition, all optional and preferred features and modifications of the described embodiments are usable in all aspects of the disclosure taught herein. Furthermore, the individual features of the dependent claims, as well as all optional and preferred features and modifications of the described embodiments are combinable and interchangeable with one another.

### BRIEF DESCRIPTION OF THE DRAWINGS

Many aspects of the present disclosure can be better understood with reference to the following drawings. The components in the drawings are not necessarily to scale, emphasis instead being placed upon clearly illustrating the principles of the present disclosure. Moreover, in the drawings, like reference numerals designate corresponding parts throughout the several views.
**FIG. 1** shows a device as described herein as a layered film structure.
**FIG. 2** shows the direct correlation established between polymer mass and resulting film thickness (with volume kept constant).
**FIG. 3** shows the erosion time of polymer films can be tuned based on the film thickness.
**FIG. 4A** shows rhodamine B release profile from cellulose acetate phthalate-pluronic F-127 (CAPP) films of thicknesses varying from 0.1-0.4 mm. **FIG. 4B** shows 5HT_{2A} agonist release from 0.1 mm CAPP films.
**FIG. 5** shows a schematic of multilayered films for Q72 pulsatile release.
**FIG. 6** shows absorption of water in CAPP films of varying CAP:P ratios.
**FIG. 7** shows erosion time of CAPP films according to different thicknesses and CAP:P ratios.
**FIG. 8** shows drug release kinetics from multilayered CAPP films demonstrate Q72 pulsatile release.
**FIGs. 9A-9B** show design (**FIG. 9A**) and drug release kinetics (**FIG. 9B**) from second generation multilayered CAPP films demonstrate consistent intermittent release profiles.
**FIG. 10** shows a schematic *in vitro* assay to assess 5HT_{2A} against bioactivity.
**FIG. 11**shows bioactivity of 5HT_{2A} agonists, both "fresh" and liberated from CAPP films.
**FIGs. 12A-12B** show a schematic **(****FIG. 12A****)** and results **(****FIG. 12B****)** of pilot pharmacokinetics study investigating the release of 2,5-dimethoxy-4-iodoamphetamine (DOI) from CAPP films *in vivo.*
**FIGs. 13A-13F** show plasma and organ pharmacokinetics of DOI delivered from subcutaneously implanted 0.1 mm thick CAPP films. **FIG. 13A****:** Study protocol. **FIG. 13B****:** DOI mass within 0.1 mm thick films. **FIG. 13C****:** Plasma, **FIG. 13D****:** Brain, **FIG. 13E****:** Liver, and **FIG. 13F****:** Kidney pharmacokinetics.
**FIGs. 14A-14D** demonstrate pulsed delivery of DOI from multilayer CAPP films *in vivo.* Quantification of DOI concentration in the **(****FIG. 14A****)** plasma, **(****FIG. 14B****)** brain, **(****FIG. 14C****)** liver, and **(****FIG. 14D****)** kidneys over time after implantation of multilayer CAPP films with the following structure: Layer 1 - 0.1 mm thick, 70:30 CAP:P with 15 ug of DOI, Layers 2 and 3 - 0.4 mm thick, 90:10 CAP:P, Layer 4 - 0.1 mm thick, 70:30 CAP:P with 15 ug of DOI, Layers 5 and 6 - 0.4 mm thick, 90:10 CAP:P, Layer 7 - 0.1 mm thick, 70:30 CAP:P with 15 ug of DOI.

Additional advantages of the invention will be set forth in part in the description which follows, and in part will be obvious from the description, or can be learned by practice of the invention. The advantages of the invention will be realized and attained by means of the elements and combinations particularly pointed out in the appended claims. It is to be understood that both the foregoing general description and the following detailed description are exemplary and explanatory only and are not restrictive of the invention, as claimed.

### DETAILED DESCRIPTION

In one aspect, disclosed herein is a method for the pulsed delivery of a 5HT_{2A} agonist to a subject, the method including at least the steps of administering to the subject a device composed of a layer structure including:
(a) a first layer including a first biodegradable polymer, wherein the first layer has a first side and a second side, and wherein the first layer comprises the 5HT_{2A} agonist;
(b) a second layer including a second biodegradable polymer, wherein the second layer has a first side and a second side, wherein the first side of the second layer is adjacent to the second side of the first layer, and wherein the second layer does not include the 5HT_{2A} agonist;
(c) a third layer including a third biodegradable polymer, wherein the third layer has a first side and a second side, wherein the first side of the third layer is adjacent to the second side of the second layer, and wherein the third layer comprises the 5HT_{2A} agonist;
(d) a fourth layer including a fourth biodegradable polymer, wherein the fourth layer has a first side and a second side, wherein the first side of the fourth layer is adjacent to the second side of the third layer, and wherein the fourth layer does not include the 5HT_{2A} agonist; and
(e) a fifth layer including a fifth biodegradable polymer, wherein the fifth layer has a first side and a second side, wherein the first side of the fifth layer is adjacent to the second side of the fourth layer, and wherein the fifth layer comprises the 5HT_{2A} agonist,
(f) wherein the device is coated with a sixth biodegradable polymer such that every surface of the device is covered with the sixth biodegradable polymer with the exception of the first side of the first layer.

In some aspects, additional pairs of layers are also included, such that stacks of layers are formed wherein layers containing 5HT_{2A} agonists alternate with layers that do not include 5HT_{2A} agonists. Many modifications and other embodiments disclosed herein will come to mind to one skilled in the art to which the disclosed compositions and methods pertain having the benefit of the teachings presented in the foregoing descriptions and the associated drawings. Therefore, it is to be understood that the disclosures are not to be limited to the specific embodiments disclosed and that modifications and other embodiments are intended to be included within the scope of the appended claims. The skilled artisan will recognize many variants and adaptations of the aspects described herein. These variants and adaptations are intended to be included in the teachings of this disclosure and to be encompassed by the claims herein.

Although specific terms are employed herein, they are used in a generic and descriptive sense only and not for purposes of limitation.

Any recited method can be carried out in the order of events recited or in any other order that is logically possible. That is, unless otherwise expressly stated, it is in no way intended that any method or aspect set forth herein be construed as requiring that its steps be performed in a specific order. Accordingly, where a method claim does not specifically state in the claims or descriptions that the steps are to be limited to a specific order, it is no way intended that an order be inferred, in any respect. This holds for any possible non-express basis for interpretation, including matters of logic with respect to arrangement of steps or operational flow, plain meaning derived from grammatical organization or punctuation, or the number or type of aspects described in the specification.

While aspects of the present disclosure can be described and claimed in a particular statutory class, such as the system statutory class, this is for convenience only and one of skill in the art will understand that each aspect of the present disclosure can be described and claimed in any statutory class.

It is also to be understood that the terminology used herein is for the purpose of describing particular aspects only and is not intended to be limiting. Unless defined otherwise, all technical and scientific terms used herein have the same meaning as commonly understood by one of ordinary skill in the art to which the disclosed compositions and methods belong. It will be further understood that terms, such as those defined in commonly used dictionaries, should be interpreted as having a meaning that is consistent with their meaning in the context of the specification and relevant art and should not be interpreted in an idealized or overly formal sense unless expressly defined herein.

Prior to describing the various aspects of the present disclosure, the following definitions are provided and should be used unless otherwise indicated. Additional terms may be defined elsewhere in the present disclosure.

### Definitions

As used herein, "comprising" is to be interpreted as specifying the presence of the stated features, integers, steps, or components as referred to, but does not preclude the presence or addition of one or more features, integers, steps, or components, or groups thereof. Moreover, each of the terms "by," "comprising," "comprises," "comprised of," "including," "includes," "included," "involving," "involves," "involved," and "such as" are used in their open, non-limiting sense and may be used interchangeably. Further, the term "comprising" is intended to include examples and aspects encompassed by the terms "consisting essentially of" and "consisting of." Similarly, the term "consisting essentially of" is intended to include examples encompassed by the term "consisting of.

As used in the specification and the appended claims, the singular forms "a," "an" and "the" include plural referents unless the context clearly dictates otherwise. Thus, for example, reference to "a bioactive agent" includes, but are not limited to, combinations or mixtures of two or more such bioactive agents, and the like.

It should be noted that ratios, concentrations, amounts, and other numerical data can be expressed herein in a range format. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. Ranges can be expressed herein as from "about" one particular value, and/or to "about" another particular value. Similarly, when values are expressed as approximations, by use of the antecedent "about," it will be understood that the particular value forms a further aspect. For example, if the value "about 10" is disclosed, then "10" is also disclosed.

When a range is expressed, a further aspect includes from the one particular value and/or to the other particular value. For example, where the stated range includes one or both of the limits, ranges excluding either or both of those included limits are also included in the disclosure, e.g. the phrase "x to y" includes the range from 'x' to 'y' as well as the range greater than 'x' and less than 'y.' The range can also be expressed as an upper limit, e.g. 'about x, y, z, or less' and should be interpreted to include the specific ranges of 'about x,' 'about y', and 'about z' as well as the ranges of 'less than x', less than y', and 'less than z'. Likewise, the phrase 'about x, y, z, or greater' should be interpreted to include the specific ranges of 'about x,' 'about y,' and 'about z' as well as the ranges of 'greater than x,' greater than y,' and 'greater than z.' In addition, the phrase "about 'x' to 'y'", where 'x' and 'y' are numerical values, includes "about 'x' to about 'y'".

It is to be understood that such a range format is used for convenience and brevity, and thus, should be interpreted in a flexible manner to include not only the numerical values explicitly recited as the limits of the range, but also to include all the individual numerical values or sub-ranges encompassed within that range as if each numerical value and sub-range is explicitly recited. To illustrate, a numerical range of "about 0.1% to 5%" should be interpreted to include not only the explicitly recited values of about 0.1% to about 5%, but also include individual values (e.g., about 1%, about 2%, about 3%, and about 4%) and the sub-ranges (e.g., about 0.5% to about 1.1%; about 5% to about 2.4%; about 0.5% to about 3.2%, and about 0.5% to about 4.4%, and other possible sub-ranges) within the indicated range.

As used herein, the terms "about," "approximate," "at or about," and "substantially" mean that the amount or value in question can be the exact value or a value that provides equivalent results or effects as recited in the claims or taught herein. That is, it is understood that amounts, sizes, formulations, parameters, and other quantities and characteristics are not and need not be exact, but may be approximate and/or larger or smaller, as desired, reflecting tolerances, conversion factors, rounding off, measurement error and the like, and other factors known to those of skill in the art such that equivalent results or effects are obtained. In some circumstances, the value that provides equivalent results or effects cannot be reasonably determined. In such cases, it is generally understood, as used herein, that "about" and "at or about" mean the nominal value indicated ±10% variation unless otherwise indicated or inferred. In general, an amount, size, formulation, parameter or other quantity or characteristic is "about," "approximate," or "at or about" whether or not expressly stated to be such. It is understood that where "about," "approximate," or "at or about" is used before a quantitative value, the parameter also includes the specific quantitative value itself, unless specifically stated otherwise.

As used herein, the terms "optional" or "optionally" means that the subsequently described event or circumstance can or cannot occur, and that the description includes instances where said event or circumstance occurs and instances where it does not.

As used herein, the term "admixing" is defined as mixing two or more components together so that there is no chemical reaction or physical interaction. The term "admixing" also includes the chemical reaction or physical interaction between the two or more components.

As used herein, the terms "treating" and "treatment" can refer generally to obtaining a desired pharmacological and/or physiological effect. The effect can be, but does not necessarily have to be, prophylactic in terms of preventing or partially preventing a disease, symptom, or condition thereof, such as depression, anxiety, obsessive compulsive disorder (OCD), addiction, or any combination thereof. The effect can be therapeutic in terms of a partial or complete cure of a disease, condition, symptom, or adverse effect attributed to the disease, disorder, or condition. The term "treatment" as used herein can include any treatment of depression, anxiety, OCD, addiction, or another neuropsychiatric disease in a subject, particularly a human and can include any one or more of the following: (a) preventing the disease from occurring in a subject which may be predisposed to the disease but has not yet been diagnosed as having it; (b) inhibiting the disease, i.e., arresting its development; and (c) relieving the disease, i.e., mitigating or ameliorating the disease and/or its symptoms or conditions. The term "treatment" as used herein can refer to both therapeutic treatment alone, prophylactic treatment alone, or both therapeutic and prophylactic treatment. Those in need of treatment (subjects in need thereof) can include those already with the disorder and/or those in which the disorder is to be prevented. As used herein, the term "treating", can include inhibiting the disease, disorder, or condition, e.g., impeding its progress; and relieving the disease, disorder, or condition, e.g., causing regression of the disease, disorder and/or condition. Treating the disease, disorder, or condition can include ameliorating at least one symptom of the particular disease, disorder, or condition, even if the underlying pathophysiology is not affected, e.g., such as treating the pain of a subject by administration of an analgesic agent even though such agent does not treat the cause of the pain.

As used herein, the term "prevent" or "preventing" refers to precluding, averting, obviating, forestalling, stopping, or hindering something from happening, especially by advance action. It is understood that where reduce, inhibit, or prevent are used herein, unless specifically indicated otherwise, the use of the other two words is also expressly disclosed.

### Methods for the Pulsed Delivery of a Bioactive Agent

Described herein are methods that provide the pulsed delivery of a bioactive agent to a subject upon administration of a device to the subject. The devices are composed of biodegradable polymers such that when administered to the subject the polymers erode and release the bioactive agent at specific time intervals. The term "microdosing" is referred to as the administration of a bioactive agent in small quantities (e.g., microgram scale) every 3-7 days to treat or prevent a disease or symptom thereof.

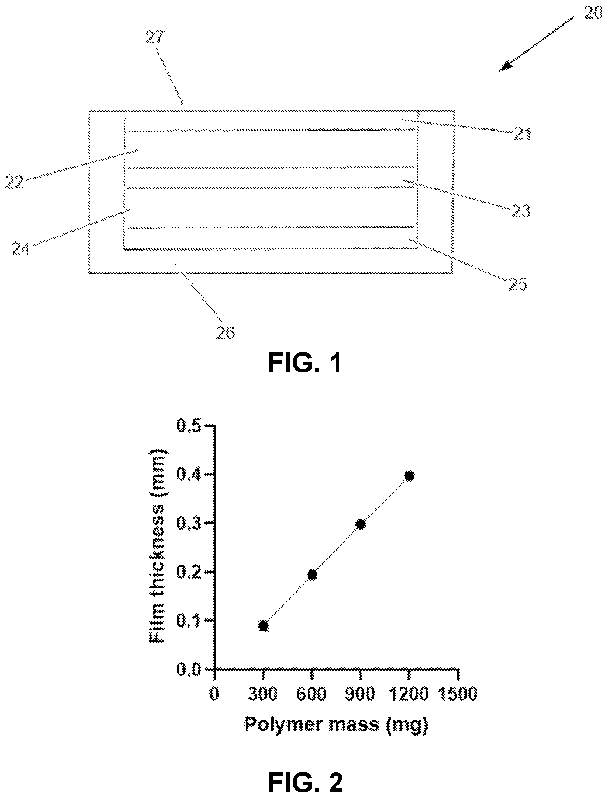

A delivery device described herein is depicted in **FIG. 1****.** The device depicted in **FIG. 1** is layered system with alternating layers of polymers that include a bioactive agent and other alternating layers that do not include the bioactive agent. The layers that do not include the bioactive agent are "blank" layers that can be designed to turn on and off delivery of the bioactive agent about every 48 hours, 72 hours, or 96 hours to generate a dosing regimen commonly used in microdosing. In one aspect, the layered films can deliver drug pulses on specified days and drug delivery will be paused by the "blank" layers on alternating days.

Referring to **FIG. 1****,** the device 20 includes three layers composed of a bioactive agent (21, 23, and 25 in **FIG. 1****)** and two layers with no bioactive agent (22 and 24 in **FIG. 1**). Each of the layers are adjacent to (i.e., in contact with) one another. The composition of the different layers can vary. In one aspect, layers 21, 23, and 25 are composed of the same biodegradable polymer. In another aspect, layers 22 and 24 are composed of the same biodegradable polymer. In another aspect, layers 21-25 are composed of the same biodegradable polymer, or are composed of different biodegradable polymers.

In one aspect, when layers 21-25 are composed of the same biodegradable polymer, the second biodegradable polymer includes a mixture of cellulose acetate phthalate (CAP) in the amount of about 50 mol% to about 90 mol% and a poloxamer in the amount of about 10 mol% to about 50 mol%. In another aspect, the second biodegradable polymer includes cellulose acetate phthalate (CAP) in the amount of about 50 mol%, 55 mol%, 60 mol%, 65 mol%, 70 mol%, 75 mol%, 80 mol%, 85 mol%, or 90 mol%, where any value can be a lower and/or upper endpoint of a range (e.g., 60 mol% to 80 mol%, etc.). In another aspect, the second biodegradable polymer includes a poloxamer in the amount of about 10 mol%, 15 mol%, 20 mol%, 25 mol%, 30 mol%, 35 mol%, 40 mol%, 45 mol%, or 50 mol%, where any value can be a lower and/or upper endpoint of a range (e.g., 20 mol% to 40 mol%, etc.).

In one aspect, the poloxamer is a nonionic triblock copolymer composed of a central hydrophobic chain of polyoxypropylene (*e*.*g*., (poly(propylene oxide)) flanked by two hydrophilic chains of polyoxyethylene (*e*.*g*., poly(ethylene oxide)). In one aspect, poloxamer has the formula

HO(C₂H₄O)_{b}(C₃H₆O)ₐ(C₂H₄O)_{b}OH

wherein a is from 10 to 100, 20 to 80, 25 to 70, or 25 to 70, or from 50 to 70; b is from 5 to 250, 10 to 225, 20 to 200, 50 to 200, 100 to 200, or 150 to 200. In another aspect, the poloxamer has a molecular weight from 2,000 to 15,000, 3,000 to 14,000, or 4,000 to 12,000. Poloxamers useful herein are sold under the tradename Pluronic^{®} manufactured by BASF. Non-limiting examples of poloxamers useful herein include, but are not limited to, those in Table 1. In one aspect, the poloxamer is F-127.

| **Table 1** | | | | |
|---|---|---|---|---|
| **Copolymer** | **MW** | **Average number of EO units** | **Average number of PO units** | **CMC (M)** |
| F68 | 8,400 | 152.73 | 28.97 | 4.8 X 10⁻⁴ |
| P103 | 4,950 | 33.75 | 59.74 | 6.1 X 10⁻⁶ |
| P105 | 6,500 | 73.86 | 56.03 | 6.2 X 10⁻⁶ |
| P123 | 5,750 | 39.2 | 69.4 | 4.4 X 10⁻⁶ |
| F127 | 12,600 | 200.45 | 65.17 | 2.8 X 10⁻⁶ |
| L121 | 4,400 | 10.00 | 68.28 | 1.1 X 10⁻⁶ |

In one aspect, the poloxamer can have a molecular weight of from about 4 kDa to about 20 kDa, or of about 12.6 kDa, or about 4, 5, 6, 7, 8, 9, 10, 11, 12, 13, 14, 15, 16, 17, 18, 19, or about 20 kDa, or a combination of any of the foregoing values, or a range encompassing any of the foregoing values. In another aspect, the poloxamer can have an average ethylene oxide content of from about 10 units to about 300 units, or of about 200.45 units, or of about 10, 50, 100, 150, 200, 250, or about 300 units, or a combination of any of the foregoing values, or a range encompassing any of the foregoing values. In another aspect, the poloxamer can have an ethylene oxide content of from about 25 units to about 100 units, or of about 65.17 units, or of about 25, 50, 75, or about 100 units, or a combination of any of the foregoing values, or a range encompassing any of the foregoing values.

By varying the thickness of the layers, it is possible to control the timing of the release of the bioactive agent from the layered system. In one aspect, the thickness of the second layer 22 and fourth layer 24 is greater than the thickness of the first layer 21, third layer 23, and fifth layer 25. In another aspect, the thickness of the first layer 21, third layer 23, and fifth layer 25 is less than about 0.2 mm, or is from about 0.05 mm to about 0.2 mm, or 0.05 mm, 0.1 mm, 0.15 mm, or 0.2 mm, where any value can be a lower and/or upper endpoint of a range (e.g., 0.05 mm to 1.5 mm, etc.). In one aspect, the first layer, the third layer, and the fifth layer can each independently erode over a period of from about 30 minutes to about 48 hours, or over a period of about 0.5, 1, 2, 3, 4, 8, 12, 16, 20, 24, 28, 32, 36, 40, 44, or about 48 hours.

In another aspect, the thickness of the second layer 22 and fourth layer 24 is greater than about 0.3 mm, or is from about 0.2 mm to about 0.5 mm, or 0.2 mm, 0.25 mm, 0.3 mm, 0.35 mm, 0.4 mm, 0.43 mm, or 0.5 mm, where any value can be a lower and/or upper endpoint of a range (e.g., 0.2 mm to 0.4 mm, etc.). In one aspect, the second layer 22 and the fourth layer 24 has a thickness such that the second layer and fourth layer each independently erode over a period of about 48 hours to about 96 hours, or about 72 hours.

The layered system as depicted in **FIG. 1** can be prepared by the sequential layering of polymer films on one another. For example, a solution of biodegradable polymer (e.g., CAP and poloxamer) dissolved in a solvent such as, for example, acetone can be applied to the surface of a substrate. The solvent will rapidly evaporate leaving a thin film of the biodegradable polymer. A second coating of the biodegradable polymer can then be applied to the dried film on the substrate. This process can be performed as many times as needed to provide the number of desired layers. The thickness of each layer can be controlled as well to modify the release rate of the bioactive agent.

After the layered system has been prepared, the system is coated on three sides with a biocompatible and slowly-degrading bioresorbable polymer (26 as depicted in **FIG. 1**). The coating ensures that drug release can only proceed from the uncoated side (27 as depicted in **FIG. 1**, the first side of the first layer), which ensures surface erosion of the films from the opening and ordered drug release. In one aspect, the layered system is coated with poly(sebacic acid), polycaprolactone (PCL), polylactic acid (PLA), or any combination thereof. In one aspect, the coating has a thickness from about 0.5 mm to about 2 mm.

In some aspects, the layered system further includes a seventh layer situated between the fifth layer and the sixth biodegradable/bioresorbable polymer, wherein the seventh layer includes a seventh biodegradable polymer. In some aspects, the seventh biodegradable polymer can be the same polymer as the second and fourth biodegradable polymers, or can be different from the second and fourth biodegradable polymers. In any of these aspects, the seventh layer can cover one surface of the fifth layer so that degradation or erosion of the fifth layer can happen from only one side, thereby ensuring release of any active compounds from the fifth layer at the same rate as release from the first and third layers.

In some aspects, the second, fourth, and/or seventh layer (if present) can further be subdivided into sub-layers. In one aspect, the second layer can include a first sub-layer and a second sub-layer, wherein each of the first and second sub-layers can comprise the second biodegradable polymer. In another aspect, the first and second sub-layers can each independently be about 0.4 mm thick.

In another aspect, the fourth layer can include a third sub-layer and a fourth sub-layer, wherein each of the third and fourth sub-layers can comprise the fourth biodegradable polymer. In another aspect, the third and fourth sub-layers can each independently be about 0.4 mm thick.

In still another aspect, the seventh layer can include a fifth sub-layer and a sixth sub-layer, wherein each of the fifth and sixth sub-layers can comprise the seventh biodegradable polymer. In another aspect, the fifth and sixth sub-layers can each independently be about 0.4 mm thick.

In any of these aspects, the device used in the disclosed methods can be a film having a thickness of from about 0.05 mm to about 2 mm, or of about 0.05, 0.1, 0.25, 0.5, 0.75, 1, 1.25, 1.5, 1.75, or about 2 mm, or a combination of any of the foregoing values, or a range encompassing any of the foregoing values. In some aspects, the sixth layer or coating can be added to the thickness of the film such that the sixth layer or coating increases the thickness of the film by an amount equal to the thickness of the sixth layer or coating (e.g. a 1 mm film with a 2 mm coating can have a total thickness of 3 mm, and the like).

The devices described herein and the methods for producing the same permit the inclusion of a variety of different types of bioactive agents. In one aspect, the bioactive agent includes an antibiotic, a pain reliever, an immune modulator, a growth factor, an enzyme inhibitor, a hormone, a messenger molecule, a cell signaling molecule, a receptor agonist, an oncolytic virus, a chemotherapy agent, a receptor antagonist, a nucleic acid, or any combination thereof.

In another aspect, the bioactive agent includes a 5HT_{2A} agonist such as, for example, 2,5-dimethoxy-4-iodoamphetamine (DOI), 1-acetyl-N,N-diethyllysergamide (ALD-52), O-acetylpsilocin (4-AcO-DMT), lysergic acid diethylamide (LSD), N1-(cyclopropylmethanoyl)-lysergic acid diethylamide (1CP-LSD), or psilocybin. The devices as described herein are capable of delivering consistent, long-term microdoses of the 5HT_{2A} agonist that will overcome major barriers of compliance, cost, and abuse. The microfuse devices described herein are an efficacious and safe way to perform microdosing by delivering the bioactive agent about every 48 hours to about 96 hours, or about 72 hours over many months.

The devices described herein can be administered to a subject using techniques known in the art. In one aspect, the device is implanted in the subject. In one aspect, a clinician can subcutaneously inject the device instead of performing a minor surgical procedure. This streamlined implantation procedure will save time and money and increase patient compliance even further. In another aspect, the devices described herein can be formulated with a pharmaceutically-acceptable excipient suitable for injection. In any of these aspects, the subject can be in need of treatment or prevention of depression, anxiety, obsessive compulsive disorder (OCD), or addiction.

Now having described the aspects of the present disclosure, in general, the following Examples describe some additional aspects of the present disclosure.

The present disclosure can be described in accordance with the following numbered aspects, which should not be confused with the claims.

Aspect 1. A method for the pulsed delivery of a 5HT_{2A} agonist to a subject comprising administering to the subject a device comprising a layer structure comprising
(a) a first layer comprising a first biodegradable polymer, wherein the first layer has a first side and a second side, and wherein the first layer comprises the 5HT_{2A} agonist;
(b) a second layer comprising a second biodegradable polymer, wherein the second layer has a first side and a second side, wherein the first side of the second layer is adjacent to the second side of the first layer, and wherein the second layer does not include the 5HT_{2A} agonist;
(c) a third layer comprising a third biodegradable polymer, wherein the third layer has a first side and a second side, wherein the first side of the third layer is adjacent to the second side of the second layer, and wherein the third layer comprises the 5HT_{2A} agonist;
(d) a fourth layer comprising a fourth biodegradable polymer, wherein the fourth layer has a first side and a second side, wherein the first side of the fourth layer is adjacent to the second side of the third layer, and wherein the fourth layer does not include the 5HT_{2A} agonist; and
(e) a fifth layer comprising a fifth biodegradable polymer, wherein the fifth layer has a first side and a second side, wherein the first side of the fifth layer is adjacent to the second side of the fourth layer, and wherein the fifth layer comprises the 5HT_{2A} agonist,
(f) wherein the device is coated with a sixth biodegradable polymer such that every surface of the device is covered with the sixth biodegradable polymer with the exception of the first side of the first layer.

Aspect 2. The method of aspect 1, wherein the first biodegradable polymer, the second biodegradable polymer, the third biodegradable polymer, the fourth biodegradable polymer, and the fifth biodegradable polymer are different polymers.

Aspect 3. The method of aspect 1, wherein the first biodegradable polymer, the second biodegradable polymer, the third biodegradable polymer, the fourth biodegradable polymer, and the fifth biodegradable polymer are the same polymer.

Aspect 4. The method of any one of aspects 1-3, wherein the first biodegradable polymer, the third biodegradable polymer, and the fifth biodegradable polymer are the same polymer.

Aspect 5. The method of any one of aspects 1-3, wherein the second biodegradable polymer and the fourth biodegradable polymer are the same polymer.

Aspect 6. The method of any one of aspects 1-5, wherein the first biodegradable polymer, the second biodegradable polymer, the third biodegradable polymer, the fourth biodegradable polymer, and the fifth biodegradable polymer each comprise a mixture of cellulose acetate phthalate (CAP) and a poloxamer.

Aspect 7. The method of any one of aspects 1-6, wherein the first biodegradable polymer, the second biodegradable polymer, the third biodegradable polymer, the fourth biodegradable polymer, and the fifth biodegradable polymer each comprises a mixture of cellulose acetate phthalate (CAP) in the amount of about 50 mol% to about 90 mol% and a poloxamer in the amount of about 10 mol% to about 50 mol%.

Aspect 8. The method of any one of aspects 1-7, wherein the poloxamer has a molecular weight of from about 4 kDa to about 20 kDa.

Aspect 9. The method of aspect 8, wherein the poloxamer has a molecular weight of about 12.6 kDa.

Aspect 10. The method of any one of aspects 1-9, wherein the poloxamer has an average ethylene oxide content of from about 10 units to about 300 units and an average propylene oxide of from about 25 units to about 100 units.

Aspect 11. The method of aspect 10, wherein the poloxamer has an average ethylene oxide content of about 200.45 units and an average propylene oxide content of about 65.17 units.

Aspect 12. The method of any one of aspects 1-11, wherein a thickness of each of the second layer and the fourth layer is greater than a thickness of each of the first layer, the third layer, and the fifth layer.

Aspect 13. The method of any one of aspects 1-12, wherein the thickness of each of the second layer and the fourth layer is greater than about 0.3 mm.

Aspect 14. The method of any one of aspects 1-12, wherein the thickness of each of the second layer and the fourth layer is about 0.4 mm.

Aspect 15. The method of any one of aspects 1-14, wherein the thickness of each of the first layer, the third layer, and the fifth layer is less than about 0.2 mm.

Aspect 16. The method of any one of aspects 1-14, wherein the thickness of each of the first layer, the third layer, and the fifth layer is about 0.1 mm.

Aspect 17. The method of any one of aspects 1-16, wherein the second layer and the fourth layer each independently erode over a period of from about 48 hours to about 96 hours.

Aspect 18. The method of any one of aspects 1-17, wherein the first layer, the third layer, and the fifth layer each independently erode over a period of from about 30 minutes to about 48 hours.

Aspect 19. The method of any one of aspects 1-18, wherein the sixth biodegradable polymer comprises poly(sebacic acid), polycaprolactone (PCL), polylactic acid (PLA), or any combination thereof.

Aspect 20. The method of any one of aspects 1-19, wherein the layer structure further comprises a seventh layer situated between the fifth layer and the sixth biodegradable polymer, wherein the seventh layer comprises a seventh biodegradable polymer.

Aspect 21. The method of aspect 20, wherein the seventh biodegradable polymer is the same polymer as the second biodegradable polymer and the fourth biodegradable polymer.

Aspect 22. The method of aspect 20, wherein the seventh biodegradable polymer is a different polymer from the second biodegradable polymer and the fourth biodegradable polymer.

Aspect 23. The method of any one of aspects 1-22, wherein the layer structure further comprises one or more additional pairs of layers, wherein each additional pairs of layers comprises a drugged layer comprising the 5HT2A agonist and a drug-releasing biodegradable polymer and an interface layer comprising an interface biodegradable polymer, wherein the interface layer does not include the 5HT2A agonist;
wherein a second side of the drugged layer is adjacent to a first side of the interface layer; and
wherein each additional pair of layers is situated in a stack with the first layer, second layer, third layer, fourth layer, and fifth layer, such that layers comprising the 5HT2A agonist alternate with layers not including the 5HT2A agonist.

Aspect 24. The method of aspect 23, wherein the drug-releasing biodegradable polymer is the same polymer as the first biodegradable polymer, the third biodegradable polymer, the fifth biodegradable polymer, or any combination thereof.

Aspect 25. The method of aspect 23 or 24, wherein interface biodegradable polymer is the same as the second biodegradable polymer, the fourth biodegradable polymer, or both the second biodegradable polymer and the fourth biodegradable polymer.

Aspect 26. The method of any one of aspects 1-25, wherein the second layer comprises a first sub-layer and a second sub-layer.

Aspect 27. The method of aspect 26, wherein the first sub-layer and the second sub-layer each comprise the second biodegradable polymer.

Aspect 28. The method of aspect 26 or 27, wherein the first sub-layer and the second sub-layer are independently each about 0.4 mm thick.

Aspect 29. The method of any one of aspects 1-28, wherein the fourth layer comprises a third sub-layer and a fourth sub-layer.

Aspect 30. The method of aspect 28 or 29, wherein the third sub-layer and the fourth sub-layer each comprise the fourth biodegradable polymer.

Aspect 31. The method of any one of aspects 1-30, wherein the third sub-layer and the fourth sub-layer are independently each about 0.4 mm thick.

Aspect 32. The method of any one of aspects 2-31, wherein the seventh layer comprises a fifth sub-layer and a sixth sub-layer.

Aspect 33. The method of aspect 32, wherein the fifth sub-layer and the sixth sub-layer each comprise the seventh biodegradable polymer.

Aspect 34. The method of aspect 32 or 33, wherein the fifth sub-layer and the sixth sub-layer are independently each about 0.4 mm thick.

Aspect 35. The method of any one of aspects 1-34, wherein the device is a film having a thickness of from about 0.05 mm to about 2 mm.

Aspect 36. The method in any one of aspects 1-35, wherein the 5HT_{2A} agonist comprises 2,5-dimethoxy-4-iodoamphetamine (DOI), 1-acetyl-N,N-diethyllysergamide (ALD-52), O-acetylpsilocin (4-AcO-DMT), lysergic acid diethylamide (LSD), N1-(cyclopropylmethanoyl)-lysergic acid diethylamide (1CP-LSD), psilocybin, or any combination thereof.

Aspect 37. The method in any one of aspects 1-36, wherein the subject is in need of treatment or prevention of neuropsychiatric disease.

Aspect 38. The method of aspect 37, wherein the neuropsychiatric disease comprises depression, anxiety, obsessive compulsive disorder, addiction, or any combination thereof.

Aspect 39. The method in any one of aspects 1-38, wherein the device is implanted into the subject.

Aspect 40. The method in any one of aspects 1-38, wherein the device is injected into the subject.

### EXAMPLES

The following examples are put forth so as to provide those of ordinary skill in the art with a complete disclosure and description of how the compounds, compositions, articles, devices and/or methods claimed herein are made and evaluated, and are intended to be purely exemplary of the disclosure and are not intended to limit the scope of what the inventors regard as their disclosure. Efforts have been made to ensure accuracy with respect to numbers (e.g., amounts, temperature, etc.), but some errors and deviations should be accounted for. Unless indicated otherwise, parts are parts by weight, temperature is in °C or is at ambient temperature, and pressure is at or near atmospheric.

### Correlation between polymer mass and thickness of polymer films.

The optimum conditions for formation of surface-eroding Cellulose Acetate Phthalate-Pluronic F-127 (CAPP) films of varying thickness was established. After screening a variety of conditions (different mol ratios of CAP to P, solvent choice, evaporation conditions, and polymer weight percent), it was determined that CAP:P mixtures dispersed well at 7% w/v in acetone and 70:30 mol ratio CAP:P films evaporated from acetone at 4 °C to form uniform and rigid films. Using Teflon(PTFE)-coated dishes with uniform diameter, films from 300, 600, 900, and 1200 mg of CAPP polymer mixture were produced. These produced films of ~0.1, 0.2, 0.3, and 0.4 mm, respectively **(****FIG. 2****).** Thus, there was a strong linear correlation between polymer mass and thickness of the films, enabling fabrication of consistent films moving forward.

### Establishment of erosion time of polymer films and the correlation between film thickness and erosion time.

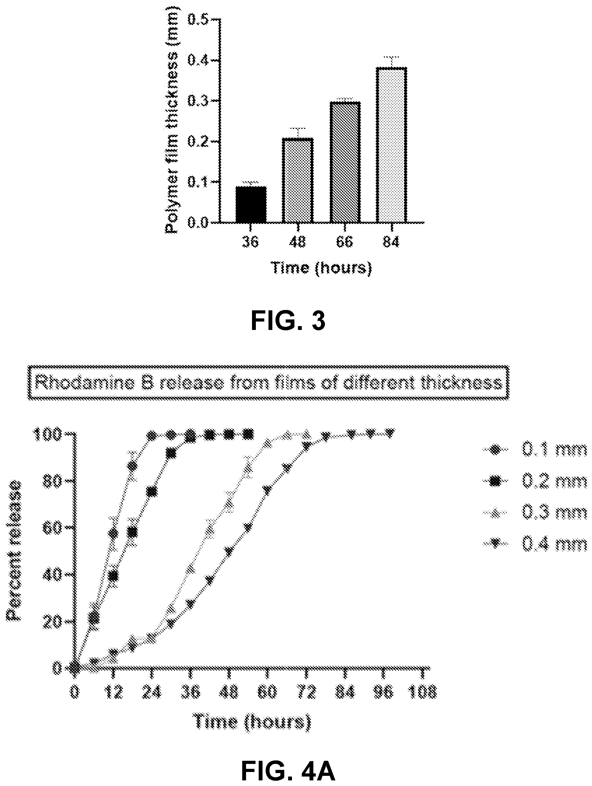

The next goal - knowing the predictable thicknesses of films based on polymer weight - was to establish any correlation between film thickness and erosion time. First, it was confirmed that the CAPP films do degrade via surface erosion, as they maintain similar mechanical properties and decrease in size as degradation occurs. Moreover, erosion time was dependent on the thickness of the films, where 0.1, 0.2, 0.3, and 0.4 mm thick films were fully eroded at 36, 48, 66, and 84 hrs, respectively **(****FIG. 3****).**

### Quantification of drug release from films of varying thickness using fluorescent dyes as drug surrogates.

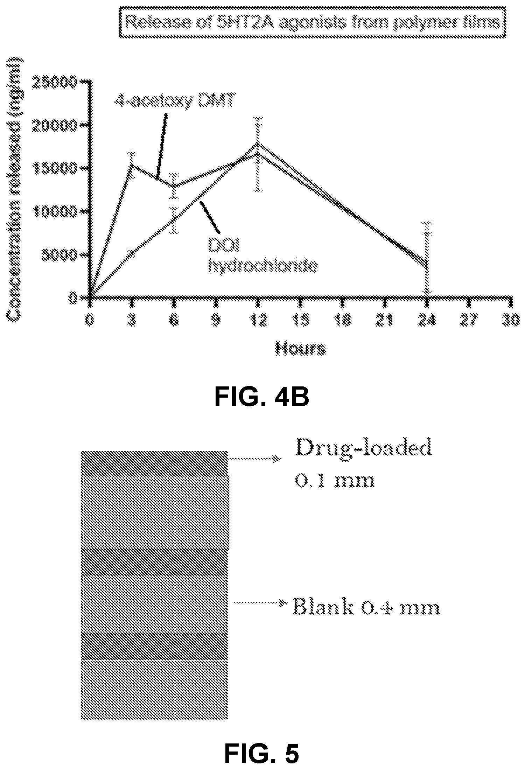

Next, drug release from single-layered CAPP films of 0.1, 0.2, 0.3, and 0.4 mm thickness was quantified. Drug release was measured by encapsulating Rhodamine fluorescent dye within the films and closely matched the erosion times reported above. Complete release from 0.1 mm thick films occurred after ~ 18 hrs (**FIG. 4A**). Complete release from 0.2 mm thick films occurred after ~ 36 hrs (**FIG. 4A**). Complete release from 0.3 mm thick films occurred after ~ 60 hrs **(****FIG. 4A**). Complete release from 0.4 mm thick films occurred after ~ 78 hrs (**FIG. 4A**). Importantly, release of 5HT_{2A} agonists from 0.1 mm thick CAPP films was also measured, since 0.1 mm thick films are used to encapsulate drug in the multilayered films. The 5HT_{2A} agonists 4-acetoxy DMT and DOI were released from 0.1 mm thick CAPP films at a very similar rate to Rhodamine dye that was used to optimize the film conditions **(****FIG. 4B****).**

### Demonstration of pulsatile release from layered films.

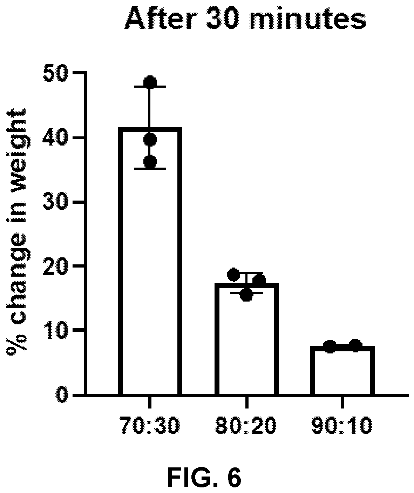

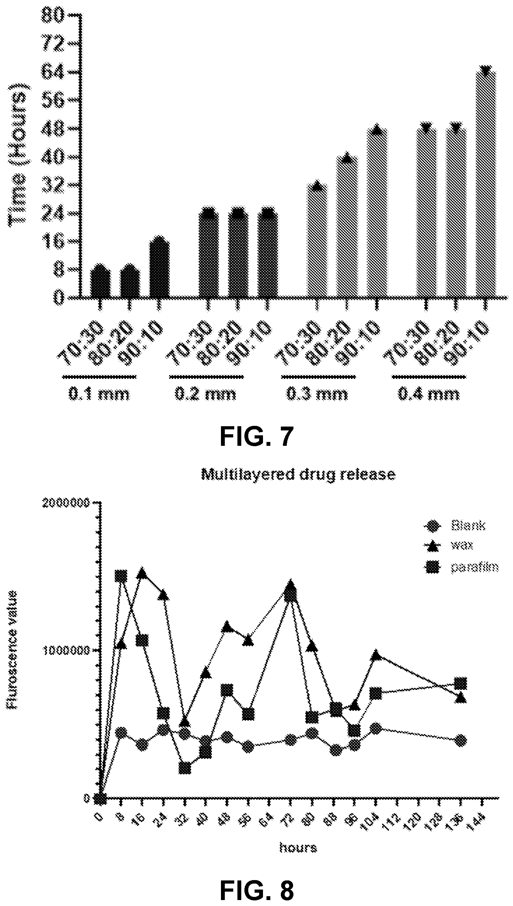

In order to achieve pulsatile release with a Q72 profile, multilayered films were constructed as shown in **FIG. 5**. The layering strategy (*i.e*., alternating 0.1 mm and 0.4 mm films) was chosen based on the results in **FIGs. 4A-4B** showing that 0.1 mm films erode in ~24 hrs while 0.4 mm films erode in ~70-80 hrs. Additionally, each layer was engineered to degrade by either bulk or surface erosion based on the function of that particular layer. The drug-loaded, 0.1 mm thick layers should degrade via bulk erosion such that the drug is rapidly released from these layers in a bolus. However, the "blank" 0.4 mm layers should erode via surface erosion to ensure gradual degradation of the blank layer from "top-to-bottom" to maintain ordered pulses of release over time. Therefore, the absorption of water into polymeric films composed of a range of CAP:P ratios was examined, since bulk erosion occurs in materials where the rate of water absorption is greater than the degradation rate. By contrast, surface erosion occurs in materials where the rate of degradation is greater than the rate of water absorption into the material. By incubating films composed of a range of CAP:P ratios varying from 70:30 to 90:10 in PBS, it was observed that water rapidly absorbed into films composed of 70:30 and 80:20 ratios of CAP:P and increased the mass of the films **(****FIG. 6****).** Alternatively, CAP:P films at a 90:10 ratio excluded water and resisted mass increase over time **(****FIG. 6****).** These data indicate that films of 70:30 and 80:20 CAP:P ratios will degrade via bulk erosion, whereas films of 90:10 CAP:P ratio will degrade via surface erosion. Therefore, multilayered films with 0.1 mm drug-loaded layers composed of 70:30 (bulk-eroding) CAPP films and 0.4 mm blank layers composed of 90:10 (surface eroding) CAPP films were produced. Lastly, the erosion time for CAPP films of CAP:P ratios varying from 70:30 - 90:10 was examined to inform the construction of multilayer devices that are to be composed of both 70:30 and 90:10 CAPP films. **FIG. 7** shows that there is a clear correlation between film thickness and erosion time, as well as a correlation between CAP:P ratio and erosion time. As film thickness increases, erosion time of the films increases proportionally. Although 70:30 and 80:20 CAP:P ratio films behave similarly, 90:10 ratio films exclude enough water to significantly increase erosion time.

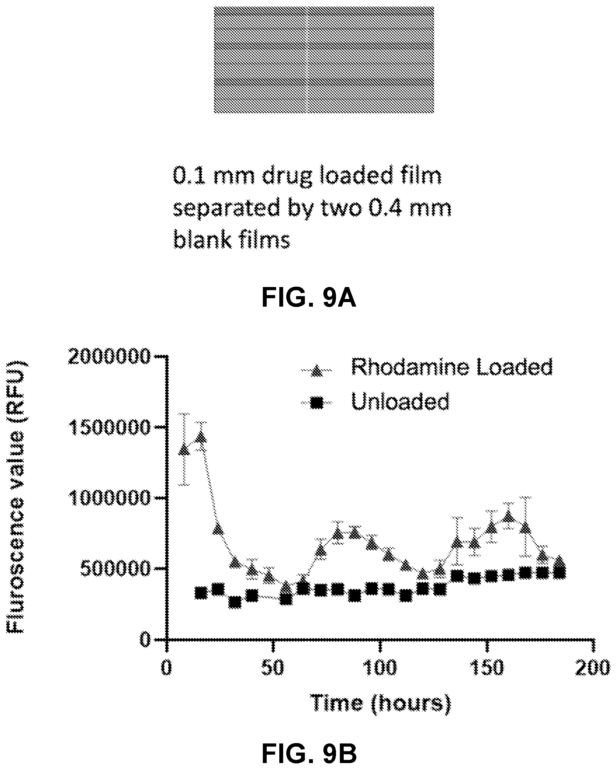

We constructed multilayer CAPP films of alternating 0.1 mm bulk eroding, drug-loaded layers and 0.4 mm surface eroding, blank layers and observed drug release kinetics from these films using Rhodamine as a model drug. As shown in **FIG.8****,** these multilayered films demonstrate intermittent release of Rhodamine with distinct drug "pulses" that peak at 16 hrs, 72 hrs, and ~120 hrs. However, in these preliminary results the third drug pulse is not obvious and the gaps between pulses have some variability. A new layering design was implemented to counter these shortcomings; including adding two "blank" layers between each drug-loaded film, as well as having "blank" layers on the bottom of the device to maintain device structure through the third drug pulse. The updated design and results of Rhodamine release from this multilayer film are shown in **FIGs. 9A-9B****.** These results demonstrate an optimized design capable of delivering three consistent intermittent drug pulses that are spaced by ~70 hrs each.

These films can be further optimized to ensure that the maximum concentration of the first peak is more consistent in magnitude with the second and third peaks, and the studies repeated with the 5HT_{2A} agonists DOI, ALD-52, and 4-AcO-DMT. Moreover, the coating that surrounds all but one side of the devices has also been optimized, since the wax and parafilm coating used for *in vitro* studies are not appropriate for implantation *in vivo.* The wax/parafilm has been replaced with a coating of the very slowly-degrading biocompatible polymer, poly(sebacic acid).

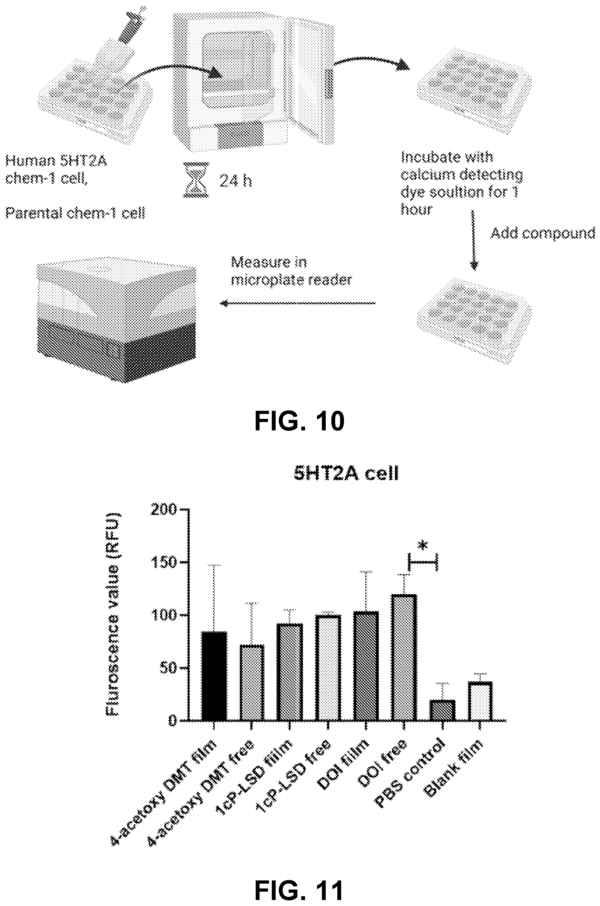

The protocol for in vitro bioactivity analysis is shown in **FIG. 10****.** Briefly, 5HT_{2A} serotonin receptor cells (Millipore Sigma, HTS082RTA) and chem-1 parental control cells (Millipore Sigma, HTSCHEM-1RTA) were seeded in 96 well plates (5000 cells - 100 µl/well of 50,000 cells/ml solution) and allowed to adhere overnight. After 24 hours, cells were treated (in serum free Opti-MEM media) with either freshly prepared 5HT_{2A} agonists (5 µM of DOI, or 4-acetoxy-DMT, or ALD-52), 5HT_{2A} agonists liberated from 0.1 mm thick CAPP films (5 µM of DOI, or 4-acetoxy-DMT, or ALD-52), dissolved "blank" CAPP films, or equal volume of PBS vehicle for 1 hr. After 1 hr, Fluo-8 green dye solution (Abcam, ab112129) was added to the cells, incubated 30 min at 37° C, and then incubated for 30 minutes at room temperature, followed by fluorescence measurements on a microplate reader.

"Blank" CAPP films did not show increased calcium flux compared to PBS control, indicating that CAPP films do not agonize the 5HT_{2A} receptor pathway (FIGs. 9A-9B). In contrast, all 5HT_{2A} agonists tested - 4-acetoxy DMT, 1cp-LSD, and DOI - showed significant induction of calcium downstream of 5HT_{2A} receptor activation. Importantly, 5HT_{2A} agonists that have been encapsulated in, and liberated from, CAPP films maintain comparable levels of 5HT_{2A} agonist activation. These results indicate that 5HT_{2A} agonists maintain their bioactivity even when encapsulated in CAPP films and delivered over time from these controlled release formulations.

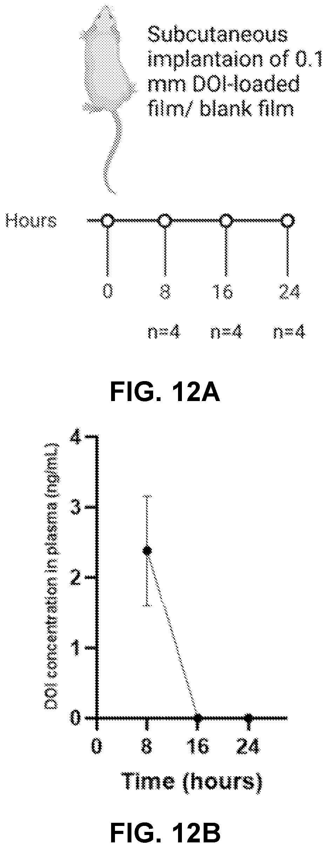

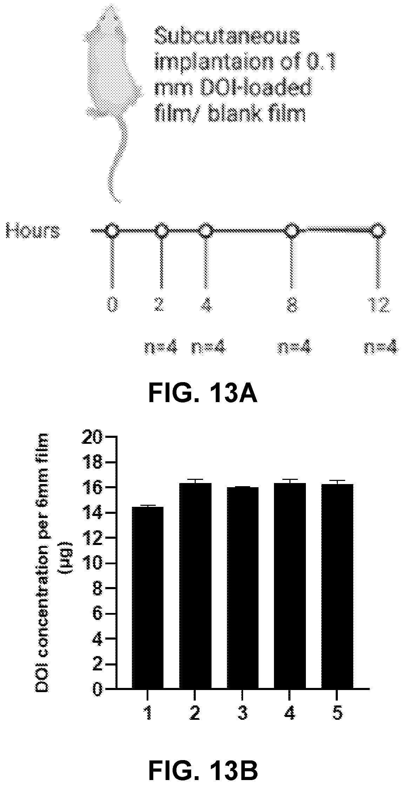

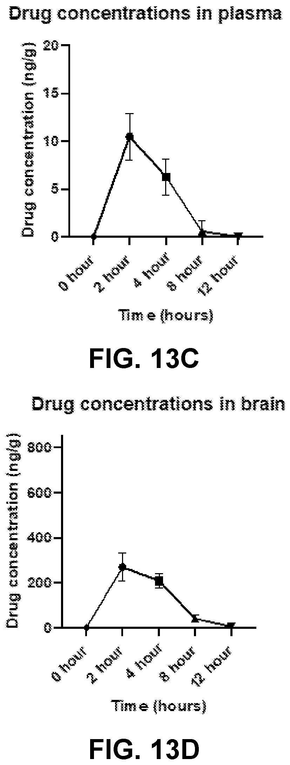

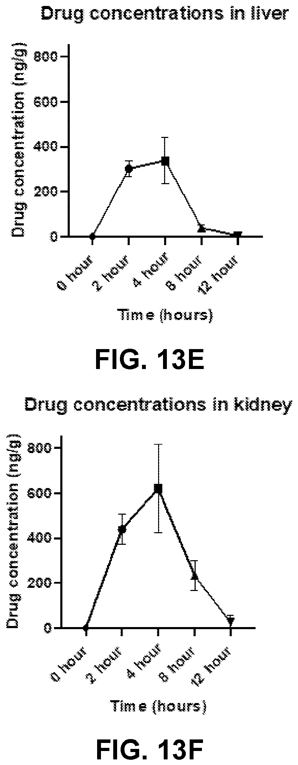

Single-layered films loaded with DOI and blank single-layered films have been constructed. These films were sterilized and implanted subcutaneously in the dorsal flap of CD-1 mice in an initial pilot experiment. This pilot experiment enabled optimization of blood collection time points, ensure the ability to detect DOI with current bioanalytical techniques, and will inform the design of the rest of the *in vivo* studies. The experimental overview of this initial pilot experiment is shown in **FIG. 12A****.** Briefly, films were implanted subcutaneously followed by blood and organ collection at 8, 16, and 24 hrs to monitor blood pharmacokinetics of DOI released from the 0.1 mm thick CAPP films in vivo. DOI was consistently detected in the blood of CD-1 mice at 8 hrs post-implantation of the DOl-loaded CAPP films (FIG. 12B). The concentration of DOI in the blood of mice returned to baseline by 16 hrs and remained at baseline through 24 hrs. These results confirm the ability to successfully implant controlled release devices and accurately quantify the blood concentration of DOI in mice post-implantation. This pilot experiment was repeated to: 1) sample at earlier time points for better overall fidelity and 2) to also determine the concentration of DOI in major organs, particularly the brain. The results from the follow-up study are shown in **FIGs. 13A-13F****.** It was found that the peak concentration of DOI in plasma occurred at 2 hrs post-implantation of 0.1 mm thick CAPP films (**FIG. 13A**). As expected based on the plasma pharmacokinetics, it was observed that the peak concentration of DOI in the brain, liver, and kidneys was ~2-4 hrs **(****FIGs. 13C-13F****).** DOI was cleared from the plasma as well as the brain, liver, and kidneys by ~12 hrs post-implantation. The release profile of the same dose of DOI from 0.4 mm thick films can also be measured, as can the release profile of DOI from multilayered CAPP films with three DOl-loaded 0.1 mm thick films separated by 0.4 mm thick blank films.

### Demonstration of Pulsed Delivery of DOI from Multilayer CAPP Films in vivo

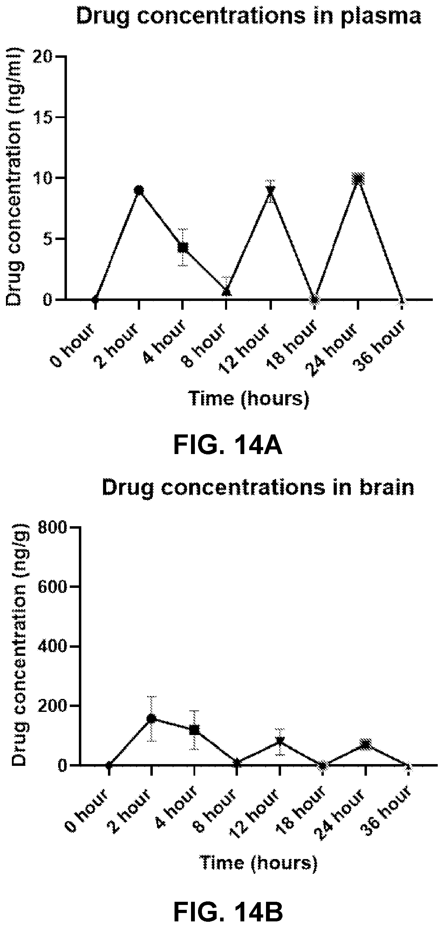

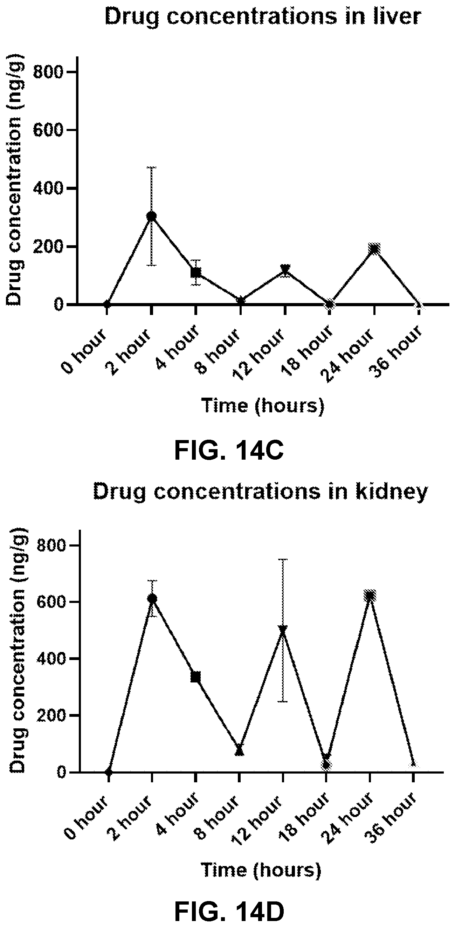

To demonstrate the ability to achieve pulsed delivery of DOI in vivo, multilayered CAPP films were implanted subcutaneously and pharmacokinetics of DOI post-implantation were monitored. The multilayered CAPP films used for this study had seven layers, where layers 1, 4, and 7 were DOl-loaded 0.1 mm thick CAPP films at 70:30 CAP:P ratio and layers 2, 3, 5, and 6 were unloaded (Blank) 0.4 mm thick CAPP films at 90:10 CAP:P ratio. The layered devices were then coated on all sides but one (the top of layer 1) with the biocompatible polymer polysebacic acid (PSA). After implantation, the anticipated pulsed release schedule of DOI was observed, with three distinct DOI pulses where peak concentrations in the plasma occurred at ~2, 12, and 24 hrs post-implantation. Moreover, the concentration of DOI in the brain, liver, and kidneys matched the pulsed profile of the blood plasma (see **FIGs. 14A-14D****).** These data highlight the ability to achieve the interval delivery of DOI in vivo from multilayered CAPP films coated in a PSA coating.

It should be emphasized that the above-described embodiments of the present disclosure are merely possible examples of implementations set forth for a clear understanding of the principles of the disclosure.

## Claims

1. A device for use in a method for the pulsed delivery of a 5HT_{2A} agonist to a subject, the method comprising administering the device to the subject,
the device comprising a layer structure comprising
(a) a first layer comprising a first biodegradable polymer, wherein the first layer has a first side and a second side, and wherein the first layer comprises the 5HT_{2A} agonist;
(b) a second layer comprising a second biodegradable polymer, wherein the second layer has a first side and a second side, wherein the first side of the second layer is adjacent to the second side of the first layer, and wherein the second layer does not include the 5HT_{2A} agonist;
(c) a third layer comprising a third biodegradable polymer, wherein the third layer has a first side and a second side, wherein the first side of the third layer is adjacent to the second side of the second layer, and wherein the third layer comprises the 5HT_{2A} agonist;
(d) a fourth layer comprising a fourth biodegradable polymer, wherein the fourth layer has a first side and a second side, wherein the first side of the fourth layer is adjacent to the second side of the third layer, and wherein the fourth layer does not include the 5HT_{2A} agonist; and
(e) a fifth layer comprising a fifth biodegradable polymer, wherein the fifth layer has a first side and a second side, wherein the first side of the fifth layer is adjacent to the second side of the fourth layer, and wherein the fifth layer comprises the 5HT_{2A} agonist,
(f) wherein the device is coated with a sixth biodegradable polymer such that every surface of the device is covered with the sixth biodegradable polymer with the exception of the first side of the first layer.

2. The device for use of claim 1, wherein the first biodegradable polymer, the second biodegradable polymer, the third biodegradable polymer, the fourth biodegradable polymer, and the fifth biodegradable polymer are different polymers, or
wherein the first biodegradable polymer, the second biodegradable polymer, the third biodegradable polymer, the fourth biodegradable polymer, and the fifth biodegradable polymer are the same polymer, or
wherein the first biodegradable polymer, the third biodegradable polymer, and the fifth biodegradable polymer are the same polymer, or
wherein the second biodegradable polymer and the fourth biodegradable polymer are the same polymer.

3. The device for use of claim 1, wherein the first biodegradable polymer, the second biodegradable polymer, the third biodegradable polymer, the fourth biodegradable polymer, and the fifth biodegradable polymer each comprise a mixture of cellulose acetate phthalate (CAP) and a poloxamer, preferably
wherein the first biodegradable polymer, the second biodegradable polymer, the third biodegradable polymer, the fourth biodegradable polymer, and the fifth biodegradable polymer each comprises a mixture of cellulose acetate phthalate (CAP) in the amount of about 50 mol% to about 90 mol% and a poloxamer in the amount of about 10 mol% to about 50 mol%, and/or
wherein the poloxamer has a molecular weight of from about 4 kDa to about 20 kDa, for example about 12.6 kDa.

4. The device for use of claim 3, wherein the poloxamer has an average ethylene oxide content of from about 10 units to about 300 units and an average propylene oxide of from about 25 units to about 100 units,
preferably, wherein the poloxamer has an average ethylene oxide content of about 200.45 units and an average propylene oxide content of about 65.17 units.

5. The device for use of claim 1, wherein a thickness of each of the second layer and the fourth layer is greater than a thickness of each of the first layer, the third layer, and the fifth layer,
preferably, wherein the thickness of each of the second layer and the fourth layer is greater than about 0.3 mm, more preferably, wherein the thickness of each of the second layer and the fourth layer is about 0.4 mm, and/or
preferably, wherein the thickness of each of the first layer, the third layer, and the fifth layer is less than about 0.2 mm, more preferably, wherein the thickness of each of the first layer, the third layer, and the fifth layer is about 0.1 mm.

6. The device for use of claim 1, wherein the second layer and the fourth layer each independently erode over a period of from about 48 hours to about 96 hours, and/or
wherein the first layer, the third layer, and the fifth layer each independently erode over a period of from about 30 minutes to about 48 hours.

7. The device for use of claim 1, wherein the sixth biodegradable polymer comprises poly(sebacic acid), polycaprolactone (PCL), polylactic acid (PLA), or any combination thereof.

8. The device for use of claim 1, wherein the layer structure further comprises a seventh layer situated between the fifth layer and the sixth biodegradable polymer, wherein the seventh layer comprises a seventh biodegradable polymer, preferably
wherein the seventh biodegradable polymer is the same polymer as the second biodegradable polymer, the fourth biodegradable polymer, or both the second biodegradable polymer and the fourth biodegradable polymer, or wherein the seventh biodegradable polymer is a different polymer from the second biodegradable polymer and the fourth biodegradable polymer, and/or
wherein the seventh layer comprises a fifth sub-layer and a sixth sub-layer,
more preferably wherein the fifth sub-layer and the sixth sub-layer each comprise the seventh biodegradable polymer and/or wherein the fifth sub-layer and the sixth sub-layer are independently each about 0.4 mm thick.

9. The device for use of claim 1, wherein the layer structure further comprises one or more additional pairs of layers, wherein each additional pairs of layers comprises a drugged layer comprising the 5HT_{2A} agonist and a drug-releasing biodegradable polymer and an interface layer comprising an interface biodegradable polymer, wherein the interface layer does not include the 5HT_{2A} agonist;
wherein a second side of the drugged layer is adjacent to a first side of the interface layer; and
wherein each additional pair of layers is situated in a stack with the first layer, second layer, third layer, fourth layer, and fifth layer, such that layers comprising the 5HT_{2A} agonist alternate with layers not including the 5HT_{2A} agonist,
preferably, wherein the drug-releasing biodegradable polymer is the same polymer as the first biodegradable polymer, the third biodegradable polymer, the fifth biodegradable polymer, or any combination thereof, and/or
preferably, wherein the interface biodegradable polymer is the same as the second biodegradable polymer, the fourth biodegradable polymer, or both the second biodegradable polymer and the fourth biodegradable polymer.

10. The device for use of claim 1,
wherein the second layer comprises a first sub-layer and a second sub-layer,
preferably, wherein the first sub-layer and the second sub-layer each comprise the second biodegradable polymer, and/or
preferably, wherein the first sub-layer and the second sub-layer are independently each about 0.4 mm thick,
and/or
wherein the fourth layer comprises a third sub-layer and a fourth sub-layer,
preferably, wherein the third sub-layer and the fourth sub-layer each comprise the fourth biodegradable polymer, and/or
preferably, wherein the third sub-layer and the fourth sub-layer are independently each about 0.4 mm thick.

11. The device for use of claim 1, wherein the device is a film having a thickness of from about 0.05 mm to about 2 mm.

12. The device for use of any one of claims 1-11, wherein the 5HT_{2A} agonist comprises 2,5-dimethoxy-4-iodoamphetamine (DOI), 1-acetyl-N,N-diethyllysergamide (ALD-52), O-acetylpsilocin (4-AcO-DMT), lysergic acid diethylamide (LSD), N1-(cyclopropylmethanoyl)-lysergic acid diethylamide (1CP-LSD), psilocybin, or any combination thereof.

13. The device for use of any one of claims 1-12, wherein the subject is in need of treatment or prevention of neuropsychiatric disease,
preferably, wherein the neuropsychiatric disease comprises depression, anxiety, obsessive compulsive disorder, addiction, or any combination thereof.

14. The device for use of any one of claims 1-13, wherein the method comprises implanting or injecting the device into the subject.

## Patentansprüche

1. Vorrichtung zur Verwendung in einem Verfahren zur gepulsten Abgabe eines 5HT_{2A}-Agonisten an ein Subjekt, wobei das Verfahren das Verabreichen der Vorrichtung an das Subjekt umfasst,
wobei die Vorrichtung eine Schichtstruktur umfasst, die umfasst:
(a) eine erste Schicht, die ein erstes biologisch abbaubares Polymer umfasst, wobei die erste Schicht eine erste Seite und eine zweite Seite aufweist und wobei die erste Schicht den 5HT_{2A}-Agonisten umfasst;
(b) eine zweite Schicht, die ein zweites biologisch abbaubares Polymer umfasst, wobei die zweite Schicht eine erste Seite und eine zweite Seite aufweist, wobei die erste Seite der zweiten Schicht zu der zweiten Seite der ersten Schicht benachbart ist und wobei die zweite Schicht den 5HT_{2A}-Agonisten nicht einschließt;
(c) eine dritte Schicht, die ein drittes biologisch abbaubares Polymer umfasst, wobei die dritte Schicht eine erste Seite und eine zweite Seite aufweist, wobei die erste Seite der dritten Schicht zu der zweiten Seite der zweiten Schicht benachbart ist und wobei die dritte Schicht den 5HT_{2A}-Agonisten umfasst;
(d) eine vierte Schicht, die ein viertes biologisch abbaubares Polymer umfasst, wobei die vierte Schicht eine erste Seite und eine zweite Seite aufweist, wobei die erste Seite der vierten Schicht zu der zweiten Seite der dritten Schicht benachbart ist und wobei die vierte Schicht den 5HT_{2A}-Agonisten nicht umfasst; und
(e) eine fünfte Schicht, die ein fünftes biologisch abbaubares Polymer umfasst, wobei die fünfte Schicht eine erste Seite und eine zweite Seite aufweist, wobei die erste Seite der fünften Schicht zu der zweiten Seite der vierten Schicht benachbart ist und wobei die fünfte Schicht den 5HT_{2A}-Agonisten umfasst,
(f) wobei die Vorrichtung mit einem sechsten biologisch abbaubaren Polymer beschichtet ist, derart, dass jede Oberfläche der Vorrichtung mit dem sechsten biologisch abbaubaren Polymer bedeckt ist, mit Ausnahme der ersten Seite der ersten Schicht.

2. Vorrichtung zur Verwendung nach Anspruch 1, wobei das erste biologisch abbaubare Polymer, das zweite biologisch abbaubare Polymer, das dritte biologisch abbaubare Polymer, das vierte biologisch abbaubare Polymer und das fünfte biologisch abbaubare Polymer unterschiedliche Polymere sind, oder
wobei das erste biologisch abbaubare Polymer, das zweite biologisch abbaubare Polymer, das dritte biologisch abbaubare Polymer, das vierte biologisch abbaubare Polymer und das fünfte biologisch abbaubare Polymer dasselbe Polymer sind, oder
wobei das erste biologisch abbaubare Polymer, das dritte biologisch abbaubare Polymer und das fünfte biologisch abbaubare Polymer dasselbe Polymer sind, oder
wobei das zweite biologisch abbaubare Polymer und das vierte biologisch abbaubare Polymer dasselbe Polymer sind.

3. Vorrichtung zur Verwendung nach Anspruch 1, wobei das erste biologisch abbaubare Polymer, das zweite biologisch abbaubare Polymer, das dritte biologisch abbaubare Polymer, das vierte biologisch abbaubare Polymer und das fünfte biologisch abbaubare Polymer jeweils ein Gemisch aus Celluloseacetatphthalat (CAP) und einem Poloxamer umfassen, vorzugsweise
wobei das erste biologisch abbaubare Polymer, das zweite biologisch abbaubare Polymer, das dritte biologisch abbaubare Polymer, das vierte biologisch abbaubare Polymer und das fünfte biologisch abbaubare Polymer jeweils ein Gemisch aus Celluloseacetatphthalat (CAP) in einer Menge von etwa 50 mol% bis etwa 90 mol% und einem Poloxamer in einer Menge von etwa 10 mol% bis etwa 50 mol% umfassen und/oder
wobei das Poloxamer ein Molekulargewicht von etwa 4 kDa bis etwa 20 kDa, beispielsweise etwa 12,6 kDa, aufweist.

4. Vorrichtung zur Verwendung nach Anspruch 3, wobei das Poloxamer einen mittleren Ethylenoxid-Gehalt von etwa 10 Einheiten bis etwa 300 Einheiten und einen mittleren Propylenoxid von etwa 25 Einheiten bis etwa 100 Einheiten aufweist,
vorzugsweise wobei das Poloxamer einen mittleren Ethylenoxid-Gehalt von etwa 200,45 Einheiten und einen mittleren Propylenoxid-Gehalt von etwa 65,17 Einheiten aufweist.

5. Vorrichtung zur Verwendung nach Anspruch 1, wobei eine Dicke von jeder der zweiten Schicht und der vierten Schicht größer ist als eine Dicke von jeder der ersten Schicht, der dritten Schicht und der fünften Schicht,
vorzugsweise wobei die Dicke von jeder der zweiten Schicht und der vierten Schicht größer als etwa 0,3 mm ist, mehr bevorzugt wobei die Dicke von jeder der zweiten Schicht und der vierten Schicht etwa 0,4 mm beträgt, und/oder
vorzugsweise wobei die Dicke von jeder der ersten Schicht, der dritten Schicht und der fünften Schicht kleiner als etwa 0,2 mm ist, mehr bevorzugt wobei die Dicke von jeder der ersten Schicht, der dritten Schicht und der fünften Schicht etwa 0,1 mm beträgt.

6. Vorrichtung zur Verwendung nach Anspruch 1, wobei die zweite Schicht und die vierte Schicht jeweils unabhängig über einen Zeitraum von etwa 48 Stunden bis etwa 96 Stunden erodieren und/oder
wobei die erste Schicht, die dritte Schicht und die fünfte Schicht jeweils unabhängig über einen Zeitraum von etwa 30 Minuten bis etwa 48 Stunden erodieren.

7. Vorrichtung zur Verwendung nach Anspruch 1, wobei das sechste biologisch abbaubare Polymer Poly(sebacinsäure), Polycaprolacton (PCL), Polymilchsäure (PLA) oder eine beliebige Kombination davon umfasst.

8. Vorrichtung zur Verwendung nach Anspruch 1, wobei die Schichtstruktur ferner eine siebte Schicht umfasst, die zwischen der fünften Schicht und dem sechsten biologisch abbaubaren Polymer angeordnet ist, wobei die siebte Schicht ein siebtes biologisch abbaubares Polymer umfasst, vorzugsweise
wobei das siebte biologisch abbaubare Polymer dasselbe Polymer ist wie das zweite biologisch abbaubare Polymer, das vierte biologisch abbaubare Polymer oder sowohl das zweite biologisch abbaubare Polymer als auch das vierte biologisch abbaubare Polymer, oder wobei das siebte biologisch abbaubare Polymer ein anderes Polymer als das zweite biologisch abbaubare Polymer und das vierte biologisch abbaubare Polymer ist, und/oder
wobei die siebte Schicht eine fünfte Unterschicht und eine sechste Unterschicht umfasst,
wobei vorzugsweise die fünfte Unterschicht und die sechste Unterschicht jeweils das siebte biologisch abbaubare Polymer umfassen und/oder wobei die fünfte Unterschicht und die sechste Unterschicht jeweils unabhängig etwa 0,4 mm dick sind.

9. Vorrichtung zur Verwendung nach Anspruch 1, wobei die Schichtstruktur ferner ein oder mehrere zusätzliche Schichtpaare umfasst, wobei jedes zusätzliche Schichtpaar eine arzneistoffhaltige Schicht, die den 5HT_{2A}-Agonisten und ein arzneistofffreisetzendes biologisch abbaubares Polymer umfasst, und eine Grenzflächenschicht, die ein Grenzflächen-biologisch abbaubares Polymer umfasst, wobei die Grenzflächenschicht den 5HT_{2A}-Agonisten nicht umfasst;
wobei eine zweite Seite der arzneistoffhaltigen Schicht zu einer ersten Seite der Grenzflächenschicht benachbart ist; und
wobei jedes zusätzliche Schichtpaar in einem Stapel mit der ersten Schicht, der zweiten Schicht, der dritten Schicht, der vierten Schicht und der fünften Schicht angeordnet ist, derart, dass Schichten, die den 5HT_{2A}-Agonisten umfassen, mit Schichten, die den 5HT_{2A}-Agonisten nicht umfassen, abwechseln,
vorzugsweise wobei das arzneistofffreisetzende biologisch abbaubare Polymer dasselbe Polymer ist wie das erste biologisch abbaubare Polymer, das dritte biologisch abbaubare Polymer, das fünfte biologisch abbaubare Polymer oder jedwede Kombination davon, und/oder
vorzugsweise wobei das Grenzflächen-biologisch abbaubare Polymer dasselbe ist wie das zweite biologisch abbaubare Polymer, das vierte biologisch abbaubare Polymer oder sowohl das zweite biologisch abbaubare Polymer als auch das vierte biologisch abbaubare Polymer.

10. Vorrichtung zur Verwendung nach Anspruch 1,
wobei die zweite Schicht eine erste Unterschicht und eine zweite Unterschicht umfasst,
vorzugsweise wobei die erste Unterschicht und die zweite Unterschicht jeweils das zweite biologisch abbaubare Polymer umfassen und/oder
vorzugsweise wobei die erste Unterschicht und die zweite Unterschicht jeweils unabhängig etwa 0,4 mm dick sind,
und/oder
wobei die vierte Schicht eine dritte Unterschicht und eine vierte Unterschicht umfasst,
vorzugsweise wobei jede der dritten Unterschicht und der vierten Unterschicht das vierte biologisch abbaubare Polymer umfassen und/oder
vorzugsweise wobei jede der dritten Unterschicht und der vierten Unterschicht unabhängig etwa 0,4 mm dick sind.

11. Vorrichtung zur Verwendung nach Anspruch 1, wobei die Vorrichtung eine Folie mit einer Dicke von etwa 0,05 mm bis etwa 2 mm ist.

12. Vorrichtung zur Verwendung nach einem der Ansprüche 1 bis 11, wobei der 5HT_{2A}-Agonist 2,5-Dimethoxy-4-iodoamphetamin (DOI), 1-Acetyl-N,N-diethyllysergamid (ALD-52), O-Acetylpsilocin (4-AcO-DMT), Lysergsäurediethylamid (LSD), N1-(Cyclopropylmethanoyl)-lysergsäurediethylamid (1CP-LSD), Psilocybin oder eine beliebige Kombination davon umfasst.

13. Vorrichtung zur Verwendung nach einem der Ansprüche 1 bis 12, wobei das Subjekt einer Behandlung oder Vorbeugung einer neuropsychiatrischen Erkrankung bedarf,
vorzugsweise wobei die neuropsychiatrische Erkrankung Depression, Angststörung, Zwangsstörung, Abhängigkeit oder eine beliebige Kombination davon umfasst.

14. Vorrichtung zur Verwendung nach einem der Ansprüche 1 bis 13, wobei das Verfahren das Implantieren oder Injizieren der Vorrichtung in das Subjekt umfasst.

## Revendications

1. Dispositif destiné à être utilisé dans un procédé d'administration pulsée d'un agoniste 5HT_{2A} à un sujet,
le procédé comprenant l'administration du dispositif au sujet,
le dispositif comprenant une structure en couches comprenant
(a) une première couche comprenant un premier polymère biodégradable, dans lequel la première couche a une première face et une seconde face, et dans lequel la première couche comprend l'agoniste 5HT_{2A} ;
(b) une deuxième couche comprenant un deuxième polymère biodégradable, dans lequel la deuxième couche a une première face et une seconde face, dans lequel la première face de la deuxième couche est adjacente à la seconde face de la première couche, et dans lequel la deuxième couche ne comporte pas l'agoniste 5HT_{2A} ;
(c) une troisième couche comprenant un troisième polymère biodégradable, dans lequel la troisième couche a une première face et une seconde face, dans lequel la première face de la troisième couche est adjacente à la seconde face de la deuxième couche, et dans lequel la troisième couche comprend l'agoniste 5HT_{2A} ;
(d) une quatrième couche comprenant un quatrième polymère biodégradable, dans lequel la quatrième couche a une première face et une seconde face, dans lequel la première face de la quatrième couche est adjacente à la seconde face de la troisième couche, et dans lequel la quatrième couche ne comporte pas l'agoniste 5HT_{2A} ;
(e) une cinquième couche comprenant un cinquième polymère biodégradable, dans lequel la cinquième couche a une première face et une seconde face, dans lequel la première face de la cinquième couche est adjacente à la seconde face de la quatrième couche, et dans lequel la cinquième couche comprend l'agoniste 5HT_{2A} ;
(f) dans lequel le dispositif est recouvert d'un sixième polymère biodégradable de telle sorte que chaque surface du dispositif soit recouverte du sixième polymère biodégradable à l'exception de la première face de la première couche.

2. Dispositif destiné à être utilisé selon la revendication 1, dans lequel le premier polymère biodégradable, le deuxième polymère biodégradable, le troisième polymère biodégradable, le quatrième polymère biodégradable et le cinquième polymère biodégradable sont des polymères différents, ou
dans lequel le premier polymère biodégradable, le deuxième polymère biodégradable, le troisième polymère biodégradable, le quatrième polymère biodégradable et le cinquième polymère biodégradable sont le même polymère, ou
dans lequel le premier polymère biodégradable, le troisième polymère biodégradable et le cinquième polymère biodégradable sont le même polymère, ou
dans lequel le deuxième polymère biodégradable et le quatrième polymère biodégradable sont le même polymère.

3. Dispositif destiné à être utilisé selon la revendication 1, dans lequel le premier polymère biodégradable, le deuxième polymère biodégradable, le troisième polymère biodégradable, le quatrième polymère biodégradable et le cinquième polymère biodégradable comprennent chacun un mélange d'acétate phtalate de cellulose (CAP) et d'un poloxamère, de préférence
dans lequel le premier polymère biodégradable, le deuxième polymère biodégradable, le troisième polymère biodégradable, le quatrième polymère biodégradable et le cinquième polymère biodégradable comprennent chacun un mélange d'acétate phtalate de cellulose (CAP) en une quantité d'environ 50 % à environ 90 % mol et d'un poloxamère en une quantité d'environ 10 % à environ 50 % mol, et/ou
dans lequel le poloxamère a un poids moléculaire d'environ 4 kDa à environ 20 kDa, par exemple environ 12,6 kDa.

4. Dispositif destiné à être utilisé selon la revendication 3, dans lequel le poloxamère a une teneur moyenne en oxyde d'éthylène d'environ 10 unités à environ 300 unités et une teneur moyenne en oxyde de propylène d'environ 25 unités à environ 100 unités,
de préférence, dans lequel le poloxamère a une teneur moyenne en oxyde d'éthylène d'environ 200,45 unités et une teneur moyenne en oxyde de propylène d'environ 65,17 unités.

5. Dispositif destiné à être utilisé selon la revendication 1, dans lequel l'épaisseur de chacune de la deuxième couche et de la quatrième couche est supérieure à l'épaisseur de chacune de la première couche, de la troisième couche et de la cinquième couche,
de préférence, dans lequel l'épaisseur de chacune de la deuxième couche et de la quatrième couche est supérieure à environ 0,3 mm, de manière davantage préférée, dans lequel l'épaisseur de chacune de la deuxième couche et de la quatrième couche est d'environ 0,4 mm, et/ou de préférence, dans lequel l'épaisseur de chacune de la première couche, de la troisième couche et de la cinquième couche est inférieure à environ 0,2 mm, de manière davantage préférée, dans lequel l'épaisseur de chacune de la première couche, de la troisième couche et de la cinquième couche est d'environ 0,1 mm.

6. Dispositif destiné à être utilisé selon la revendication 1, dans lequel la deuxième couche et la quatrième couche s'érodent chacune indépendamment sur une période d'environ 48 heures à environ 96 heures, et/ou dans lequel la première couche, la troisième couche et la cinquième couche s'érodent chacune indépendamment sur une période allant d'environ 30 minutes à environ 48 heures.

7. Dispositif destiné à être utilisé selon la revendication 1, dans lequel le sixième polymère biodégradable comprend du poly(acide sébacique), de la polycaprolactone (PCL), de l'acide polylactique (PLA) ou une quelconque combinaison de ceux-ci.

8. Dispositif destiné à être utilisé selon la revendication 1, dans lequel la structure en couches comprend en outre une septième couche située entre la cinquième couche et le sixième polymère biodégradable, dans lequel la septième couche comprend un septième polymère biodégradable, de préférence
dans lequel le septième polymère biodégradable est le même polymère que le deuxième polymère biodégradable, le quatrième polymère biodégradable, ou à la fois le deuxième polymère biodégradable et le quatrième polymère biodégradable, ou dans lequel le septième polymère biodégradable est un polymère différent du deuxième polymère biodégradable et du quatrième polymère biodégradable, et/ou
dans lequel la septième couche comprend une cinquième sous-couche et une sixième sous-couche,
de manière davantage préférée dans lequel la cinquième sous-couche et la sixième sous-couche comprennent chacune le septième polymère biodégradable et/ou dans lequel la cinquième sous-couche et la sixième sous-couche ont chacune une épaisseur d'environ 0,4 mm.

9. Dispositif destiné à être utilisé selon la revendication 1, dans lequel la structure en couches comprend en outre une ou plusieurs paires de couches supplémentaires, dans lequel chaque paire de couches supplémentaires comprend une couche imprégnée de médicament comprenant l'agoniste 5HT_{2A} et un polymère biodégradable à libération de médicament et une couche d'interface comprenant un polymère biodégradable d'interface, dans lequel la couche d'interface ne comporte pas l'agoniste 5HT_{2A} ;
dans lequel une seconde face de la couche médicamenteuse est adjacente à une première face de la couche d'interface ; et
dans lequel chaque paire de couches supplémentaires est empilée avec la première couche, la deuxième couche, la troisième couche, la quatrième couche et la cinquième couche, de sorte que les couches comprenant l'agoniste 5HT_{2A} alternent avec des couches ne comportant pas l'agoniste 5HT_{2A},
de préférence, dans lequel le polymère biodégradable libérant le médicament est le même polymère que le premier polymère biodégradable, le troisième polymère biodégradable, le cinquième polymère biodégradable, ou une quelconque combinaison de ceux-ci, et/ou
de préférence, dans lequel le polymère biodégradable d'interface est le même que le deuxième polymère biodégradable, le quatrième polymère biodégradable, ou à la fois le deuxième polymère biodégradable et le quatrième polymère biodégradable.

10. Dispositif destiné à être utilisé selon la revendication 1,
dans lequel la deuxième couche comprend une première sous-couche et une deuxième sous-couche,
de préférence, dans lequel la première sous-couche et la deuxième sous-couche comprennent chacune le deuxième polymère biodégradable, et/ou
de préférence, dans lequel la première sous-couche et la deuxième sous-couche ont chacune une épaisseur indépendante d'environ 0,4 mm,
et/ou
dans lequel la quatrième couche comprend une troisième sous-couche et une quatrième sous-couche,
de préférence, dans lequel la troisième sous-couche et la quatrième sous-couche comprennent chacune le quatrième polymère biodégradable, et/ou
de préférence, dans lequel la troisième sous-couche et la quatrième sous-couche ont chacune une épaisseur indépendante d'environ 0,4 mm.

11. Dispositif destiné à être utilisé selon la revendication 1, dans lequel le dispositif est un film ayant une épaisseur d'environ 0,05 mm à environ 2 mm.

12. Dispositif destiné à être utilisé selon l'une quelconque des revendications 1 à 11, dans lequel l'agoniste 5HT_{2A} comprend la 2,5-diméthoxy-4-iodoamphétamine (DOI), le 1-acétyl-N,N-diéthyllysergamide (ALD-52), l'O-acétylpsilocine (4-AcO-DMT), le diéthylamide de l'acide lysergique (LSD), le N1-(cyclopropylméthanoyl)-diéthylamide de l'acide lysergique (1CP-LSD), la psilocybine ou une quelconque combinaison de ceux-ci.

13. Dispositif destiné à être utilisé selon l'une quelconque des revendications 1 à 12, dans lequel le sujet a besoin d'un traitement ou d'une prévention d'une maladie neuropsychiatrique,
de préférence, dans lequel la maladie neuropsychiatrique comprend la dépression, l'anxiété, les troubles obsessionnels compulsifs, la toxicomanie ou une combinaison de ceux-ci.

14. Dispositif destiné à être utilisé selon l'une quelconque des revendications 1 à 13, dans lequel le procédé comprend l'implantation ou l'injection du dispositif dans le sujet.
